# EUROPEAN PATENT APPLICATION

(11) **EP 1 437 413 A1**
(43) Date of publication of application: **14.07.2004**
(21) Application number: 02765406.0
(22) Date of filing: 03.09.2002
(51) Int. Cl.: C12N 15/85, C12P 21/00

(54) **METHOD OF EXPRESSING GENETICALLY MODIFIED PROTEIN CARRYING SUGAR CHAIN HAVING SIALIC ACID ADDED THERETO IN INSECT CELLS**

(30) Priority: 06.09.2001 JP 2001270914
(71) Applicant: Incorporated Administrative Agency, National Agriculture and Bio-Oriented Research Organization, Tsukuba-shi, Ibaraki 305-8517 (JP)
(72) Inventor: WATANABE, Satoko, Tsukuba-shi, Ibaraki 305-0044 (JP); KOKUHO, Takehiro, Tsukuba-shi, Ibaraki 305-0044 (JP); KUBOTA, Takayuki, Tsukuba-shi, Ibaraki 305-0035 (JP); INUMARU, Shigeki, Tsukuba-shi, Ibaraki 305-0042 (JP)
(74) Representative: Woods, Geoffrey Corlett
(86) International application number: PCT/JP2002/008936
(87) International publication number: WO 2003/023039

(57) **Abstract**

Provided is a method of expressing a recombinant protein having sialic acid added thereto in insect cells, which is advantageous in administration to animals. In this method, a recombinant protein carrying a sugar chain having sialic acid added thereto is expressed in an insect or insect cells, including suppressing, inhibiting or eliminating N-acetylglucosaminidase activity inherent in the insect cells.

## Description

### Technical Field

The present invention relates to a method of expressing a recombinant protein having sialic acid added thereto in insect cells, which is advantageous in administration to animals.

### Background Art

An N-linked sugar chain of a glycoprotein plays an important role in the biological activity and *in-vivo* stability of the protein. Sialic acid, which is one of the components constituting a sugar chain and which binds to an N-linked sugar chain terminus, is particularly important.

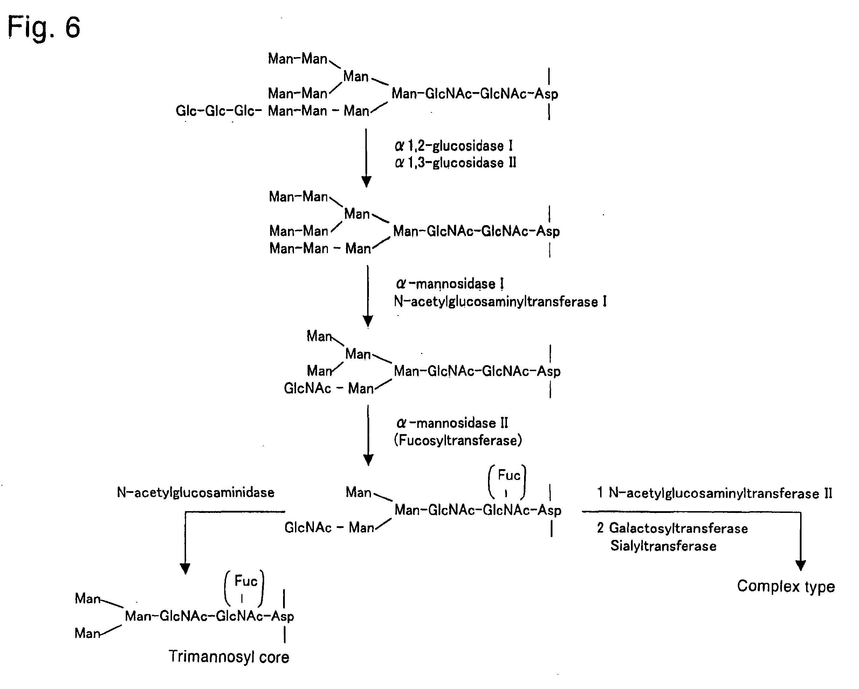

In a conventional method of expressing a recombinant protein using a baculovirus and insect cells, the N-linked sugar chain added to an expressed protein has a structure intrinsic to the insect cells, and sialylation, which is modification by the addition of sialic acid and which is frequently observed in mammals, does not occur. More specifically, in proteins expressed by use of a baculovirus-insect cell expression system, most of the N-linked sugar chains are free from sialic acid and have a trimannosyl core structure. In insect cells, N-acetylglucosaminidase (GlcNAcase), which is localized in the Golgi apparatus, has an activity of constructing the N-linked sugar chain to be added to a glycoprotein expressed in the insect cells into a structure intrinsic to the insect cells. In other words, since GlcNAcase hydrolyzes the acetylglucosamine residue at an N-linked sugar chain terminus in the synthetic pathway, conceivably, no extension of the sugar chain, to which sialic acid is to be added, takes place (Figure 6). When a recombinant protein having an insect-cell-type sugar chain is administered to an animal, it causes such problems that the insect-type sugar chain is recognized as a foreign substance or it is rarely retained in the living body for a long time. Because of these problems, even if a mammal-derived protein is expressed in insect cells, the protein has a low stability in a living animal body, so that it is deemed unsuitable for administration to animals.

### Disclosure of the Invention

The present invention provides a method of expressing a recombinant protein having sialic acid added thereto in insect cells, the recombinant protein having excellent stability *in vivo* and a structure analogous to a protein inherent in a mammal and thus suitable for administration to an animal.

The present inventors have conducted intensive studies. As a result, they found that a recombinant protein having sialic acid added thereto, which is advantageous in administration to an animal, can be obtained by suppressing, inhibiting or eliminating N-acetylglucosaminidase activity intrinsic to insect cells when the recombinant protein is expressed in an insect or insect cells. Based on the finding, the present invention has been achieved.

More specifically, the present invention is as follows:
(1) A method of expressing a recombinant protein having a sugar chain with sialic acid added thereto in an insect or insect cells, which comprises suppressing, inhibiting or eliminating N-acetylglucosaminidase activity intrinsic to insect cells.
(2) The method according to (1), in which the insect is silkworm.
(3) The method of producing a recombinant protein having a sugar chain with sialic acid added thereto according to (1), which comprises suppressing, inhibiting or eliminating N-acetylglucosaminidase activity contained in insect cells; and culturing the insect cells infected with a baculovirus having a mammal-derived protein gene introduced therein to express the protein.
(4) The method according to any one of (1) to (3), in which the suppressing, inhibiting or eliminating N-acetylglucosaminidase activity is performed by addition of an N-acetylglucosaminidase inhibitor.
(5) The method according to (4), which comprises pretreating insect cells with an N-acetylglucosaminidase inhibitor before the insect cells are infected with a baculovirus having a mammal-derived protein gene introduced therein.
(6) The method according to (4) or (5), in which the N-acetylglucosaminidase inhibitor is 2-acetoamide-1,2-dideoxynojirimycin.
(7) The method according to any one of (1) to (6), in which the protein is selected from the group consisting of interferons, granulocyte-macrophage colony-stimulating factors, and interleukins.
(8) The method according to (7), in which the protein is interferon-τ.

The present invention will be explained in detail below.

In the present invention, insect cells or an insect infected with a recombinant baculovirus having a gene encoding a protein derived from a mammal must be obtained first.

The protein derived from a mammal is not limited, however, it includes physiologically active substances, such as cytokines and growth factors, for example, interferon α, interferon β, interferon γ, interferon τ, granulocyte-macrophage colony-stimulating factors (GM-CSF), interleukins (IL1, IL2, IL3, IL4, IL5, IL6, IL7, IL8, IL9, IL10, IL11, IL12, IL13, IL15, IL17, IL18), erythropoietins (EPO), tissue plasminogen activators (TPA), granulocyte-colony stimulating factors (G-CSF), macrophage colony-stimulating factors (M-CSF), thrombopoietins (TPO), tumor necrosis factors (TNF), human chorionic gonadotropins (hCG), nerve growth factors (NGF), urokinase (UK), blood coagulation factor VIII (factor VIII), and blood coagulation factor (factor IX), derived from a human, bovine, horse, cat or dog. The recombinant baculovirus having a protein-encoding gene integrated therein can be obtained by a known method, for example, described in Molecular Biology Experimental Protocol III (published by Maruzen Co., Ltd., p665-p687, 1997). More specifically, the recombinant baculovirus can be prepared by introducing a recombinant vector having a gene encoding a desired protein to be produced in the present invention and baculovirus DNA into insect cells and causing double homologous recombination between the recombinant vector and the baculovirus DNA in the insect cells. As the baculovirus, nuclear polyhedorosis virus or the like is included.

The insect cells are not particularly limited as long as the recombinant vector according to the present invention and baculovirus DNA can be introduced. For example, Sf9 cells, Sf21 cells and TN5 cells are included.

Furthermore, when an insect is infected with the recombinant baculovirus thus obtained, an insect having a mammal-derived protein gene can be obtained in an expressible state. For example, when a silkworm is infected with the recombinant baculovirus, a silkworm capable of expressing a mammalian protein can be obtained.

When the insect cells are cultured or an insect is grown under the conditions of suppressing, inhibiting or eliminating N-acetylglucosaminidase activity, a recombinant protein having sialic acid added thereto can be produced. The conditions of suppressing, inhibiting or eliminating N-acetylglucosaminidase activity included the addition of an N-acetylglucosaminidase inhibitor. The N-acetylglucosaminidase inhibitor includes 2-acetoamide-1,2-dideoxynojirimycin (2-ADN) (p-nitorophenyl-2-deoxy-β-D-glucopyranoside). When a recombinant protein is expressed in the insect cells cultured in a medium, the concentration of the inhibitor to be added to the medium is not particularly limited; however, it is preferably several mM to ten and several mM, and more preferably 2 mM to 5 mM. The insect cells may be cultured in an appropriate culture medium, for example, EX-cell 400 medium (manufactured by JRH Biosciences) or 10% fetal bovine serum-containing TC100 medium (manufactured by GIBCO BRS Co., Ltd.), at an optimum temperature.

Even if the inhibitor is administered orally or by injection to an insect having a mammalian protein-encoding gene in an expressible state, a recombinant protein having sialic acid added thereto can be obtained.

The culturing in a medium containing an N-acetylglucosaminidase inhibitor may be performed by culturing insect cells having a mammalian protein-encoding gene in an expressible state, in a medium containing the inhibitor, in advance. However, in consideration of the addition amount of sialic acid to a recombinant protein, it is preferable that the insect cells be pretreated and then cultured in the inhibitor-containing medium before an insect is infected with a recombinant baculovirus.

When an insect having a mammalian protein-encoding gene in an expressible state is raised or insect cells having a mammalian protein-encoding gene in an expressible state are cultured, a recombinant protein having sialic acid added thereto, that is, having an animal sugar chain produced in animal cells, can be obtained from the insect thus raised or the cultured insect cells. For example, when cultured insect cells are used, a recombinant protein having sialic acid added thereto can be obtained by collecting a culture solution, a cell lysate or a homogenate of the insect cells. Insect cells may be disrupted by a method including collecting insect cells by centrifugation, suspending the insect cells in a buffer to prepare a suspension, and giving a physical impact on the suspension. As the buffer used herein, for example, TE buffer, phosphate buffer, or PBS may be used. Physical shock may be given to the suspension, for example, by ultrasonic irradiation.

Cultured insect cells may be lysed, for example, by a method including adding PBS containing about 1% of SDS or Triton X100 to the aforementioned suspension while stirring. After insect cells are disrupted or lysed, cell debris is centrifugally removed. From the obtained supernatant, a recombinant protein can be isolated and purified.

Whether or not the obtained protein has sialic acid added thereto may be determined by subjecting the protein to SDS-PAGE, transferring the protein fractions onto a PVDF membrane or a nitrocellulose membrane, and analyzing them by a blotting method using lectin. The blotting method using lectin may be performed in accordance with the method described in the glycobiology experimental protocol (Cell Engineering, Supplementary volume, 1996, edited by Naoyuki Taniguchi et al.).

### Brief Description of the Drawings

Figure 1 shows photographs indicating the results of SDS-PAGE and the results of Western blot analysis of recombinant boIFN-τ whose N-glycosylation has been inhibited (Figure 1A shows the results of SDS-PAGE and Figure 1B shows the results of Western blot).
Figure 2 shows photographs indicating the effects of the addition of a GlcNAcase inhibitor on the glycosylation of recombinant boIFN-τ (Figure 2A shows the results of SDS-PAGE and Figure 2B shows the result of Western blot).
Figure 3 shows photographs indicating the effects of 2-ADN on the production of sialylated recombinant boIFN-τ (Figure 3A shows the results of GIcNAcase inhibition assay of TN5 cells, Figure 3B shows the detection results of sialic acid in recombinant boIFN-τ, and Figure 3C shows the detection results of expressed recombinant boIFN-τ by CDD staining).
Figure 4 shows photographs indicating recombinant boIFN-τ expressed by TN5 cells is α-2,6-sialylated (Figure 4A shows the results of recombinant boIFN-τ not treated by sialidase, Figure 4B shows the results of recombinant boIFN-τ treated by sialidase, Figure 4C shows the results of recombinant boIFN-τ preincubated with 0.1 mM 6'-sialyllactose, and Figure 4D shows the results of recombinant boIFN-τ preincubated with 10 mM 3'-sialyllactose).
Figure 5 shows photographs indicating that recombinants GM-CSF and IL2 produced in insect cells are sialylated (Figure 5A shows the results of CBB staining and Figure 5B shows the results of lectin blot analysis).
Figure 6 shows the pathway of N-glycosylation in insect cells.

### Best Mode for Carrying Out the Invention

Hereinbelow, the present invention will be explained in more detail with reference to Examples, which should not be construed as limiting the technical scope of the present invention.

### [Example 1] Preparation of recombinant baculovirus having bovine interferon-T (boIFN-τ) gene introduced therein

The mRNA of bovine interferon-τ (boIFN-τ) serving as a template was obtained from bovine trophoblast cells in accordance with a method described in Takahashi, M. et al., Cloned Animal and Placentation, 146-150, (2000). Then, cDNA encoding an ORF of boIFN-τ was amplified by an RT-PCR and cloned in a plasmid vector, pCRII (manufactured by Invitrogen Corporation). A recombinant baculovirus transfer vector having cDNA of boIFN-τ and a recombinant baculovirus were constructed in accordance with the method described in Inumaru, S. et al., Immunol. Cell. Biol. 76, 195-201 (1998).

### [Example 2] Culture of insect cells containing boIFN-τ gene added with N-acetylglucosaminidase inhibitor

### (1) Infection of insect cells with baculovirus using medium containing N-acetylglucosaminidase inhibitor

TN5 cells were cultured in EX-CELL401 medium (manufactured by JRH Bioscience) containing 0 to 5 mM of 2-acetamide-1,2-dideoxynojirimycin (2-ADN) (Tront Research Chemicals Inc.) at 28°C for 3 days. Subsequently, TN5 (TN5B1-4) cells were infected with the recombinant baculovirus prepared as described above at an moi of 1.0 and subjected to a further culturing in EX-CELL401 medium (manufactured by JRH Bioscience) at 28°C for 3 days. Thereafter, the supernatant containing recombinant boIFN-τ (rboIFN-τ) was recovered.

The addition of a sugar chain to a recombinant protein, that is, N-glycosylation, was inhibited by culturing the TN5 cells infected with a recombinant baculovirus in a medium containing 1 µg/ml tunicamycin (manufactured by Sigma Chemical Co.) for 3 days.

### (2) SDS-PAGE and Western blot analyses of the culture supernatant of insect cells

Twenty µl of the culture supernatant of TN5 cells cultured for 3 days after infection with a recombinant baculovirus was subjected as a sample to Tricine SDS-PAGE performed under reducing conditions in accordance with a method described in Schagger, H. et al., Anal. Biochem. 166, 368-379 (1987). After electrophoresis, the separated protein was stained with Coomassie brilliant blue (CBB)G-250 or blotted on a polyvinylidene difluoride (PDFV) membrane (manufactured by Millipore Corporation). The protein-transferred membrane was blocked with PBS containing 5% skim milk at 4°C overnight and allowed to react with a 0.2 µg/ml anti-boIFN-τ monoclonal antibody (manufactured by Katakura Industries) at room temperature for one hour, and further incubated with 2000-fold-diluted HPR-rabbit anti-mouse IgG at room temperature. The antibody bound to the protein was visualized by 3,3'-diaminobenzidine staining. The densities of bands obtained after the CBB staining and Western blotting were quantified by using a densitometer.

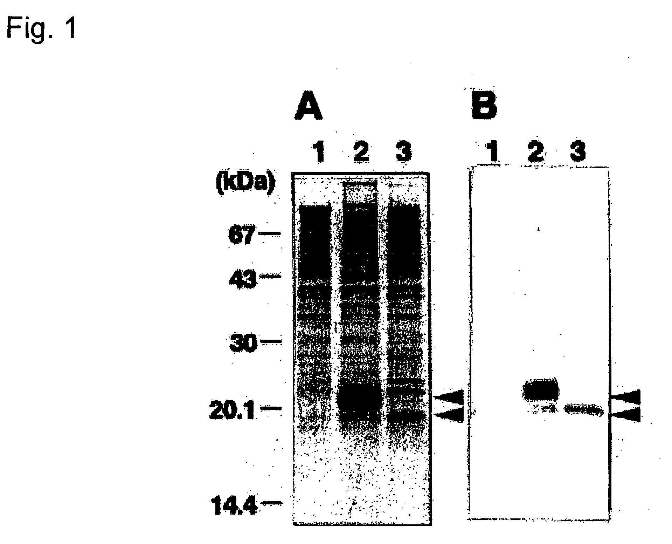

The results of SDS-PAGE and Western blotting of recombinant boIFN-τ whose N-glycosylation has been inhibited by the treatment of tunicamycin are shown in Figure 1.

In Figure 1, Lane 1 shows the analysis of TN5 cells infected with a wild-type virus and thus containing no mammalian protein-encoding gene; Lane 2 shows the analysis of TN5 cells cultured in the absence of tunicamycin and having a mammalian protein-encoding gene in an expressible state; and Lane 3 shows the analysis of TN5 cells in the presence of tunicamycin and having a mammalian protein-encoding gene in an expressible state. Proteins of about 19.5 kD and 22 kDa were observed in the culture supernatant (Figure 1A, Lane 2). These two molecules reacted with the anti-boIFN-τ monoclonal antibody (Figure 1B, Lane 2). In the case where N-glycosylation was blocked by tunicamycin, only the 19.5 kD molecule was observed in CBB staining (Figure 1A, Lane 3) and Western blot analysis (Figure 1B, Lane 3). These results demonstrate that the 22 kDa molecule is N-glycosylated and the 19.5 kDa molecule is not.

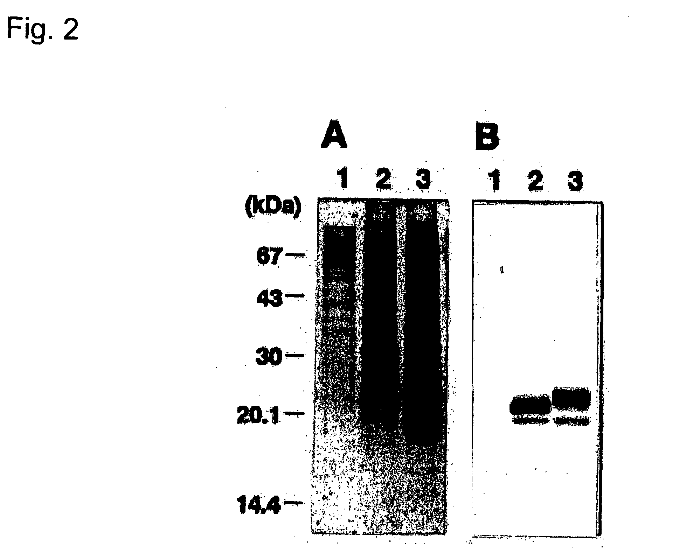

Figure 2 shows the effects of the addition of a GlcNAcase inhibitor on the glycosylation of recombinant boIFN-τ. Figure 2A shows the results of CBB staining after SDS-PAGE and Figure 2B shows the results of Western blotting after SDS-PAGE. Samples of individual lanes are as follows: Lane 1 is the culture supernatant of TN5 cells infected with a wild-type virus cultured in a medium containing 2-ADN; Lane 2 is the culture supernatant containing recombinant boIFN-τ obtained from the culture in the absence of 2-ADN; and Lane 3 is the culture supernatant containing recombinant boIFN-τ obtained from the culture in the presence of 2-ADN (5 mM). Recombinant boIFN-τ obtained in the presence of a GlcNAcase inhibitor had a molecular weight of 23.3 kDa, which was significantly larger than that of 22 kDa obtained in the absence of the inhibitor. However, although it is not shown in the figures, the molecular weight of unglycosylated recombinant boIFN-τ whose N-glycosylation was blocked by tunicamycin remained 19.5 kDa even if 2-ADN was added. These results demonstrate that the increase of N-glycosylated recombinant boIFN-τ in molecular weight is due to the increase of the sugar chain moiety, in other words, a sugar chain is modified by 2-ADN treatment.

Note that even if 2-ADN was added in a concentration of 5 mM, the proliferation of cells was not affected.

### (3) Lection blot analysis

After TN5 cells were infected with a recombinant baculovirus, they were cultured for 3 days. The supernatant was subjected to Tricine SDS-PAGE performed under reducing conditions (600 ng protein per lane). After the separated protein was transferred on a PVDF membrane, and blocked with PBS containing 1% BSA at 4°C overnight, the protein was washed with 200 mM Tris-buffered saline (TBS) containing 0.05% Tween 20, and then reacted with 5 µg/ml Sambucus nigra agglutinin labeled with horseradish peroxidase (HRP-SNA, manufactured by EY laboratory) at room temperature for one hour. The carbohydrate reacted was visualized by a Renaissance Western Blot Chemiluminescence Kit (Manufactured by NEN).

To investigate whether or not a sialylated recombinant glycoprotein is produced in insect cells where the GlcNAcase activity is inhibited, the terminal structure of N-glycan of recombinant boIFN-τ was determined by a lectin blot analysis using SNA, which is a lectin capable of recognizing α-2, 6-linked sialic acid.

The GlcNAcase inhibition assay of TN5 cells was performed as follows.

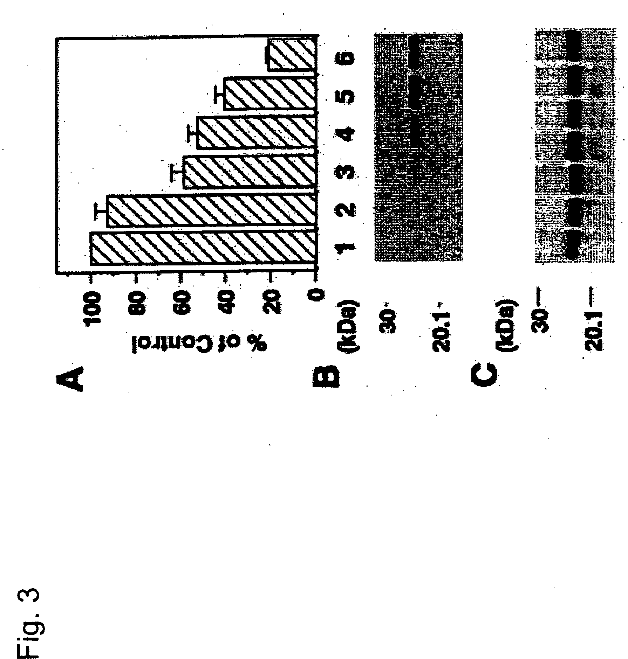

TN5 cells (2.0 x 10⁷ cells) were washed once with PBS and rinsed with 1 ml of PBS containing 1% Triton-X 100. After centrifugation at 1,000 x g for 5 minutes, the supernatant was used as an enzyme source. Fifty µl of the supernatant was mixed with 50 µl of 2-ADN to make a final 2-ADN concentration of 0 to 5 mM. To the solution mixture, 200 µl of a substrate solution (1 mg/ml p-nitrophenyl β-D-acetylglucosamide in 0.2 M citrate/phosphate buffer) was added. The resultant solution was incubated at 37°C for one hour. The reaction was quenched by adding 1 M Na₂CO₃, the absorption of released p-nitrophenol was measured at 420 nm. The results are shown in Figure 3. Figure 3A shows the results of GlcNAcase inhibition assay in TN5 cells in which the degree of inhibition is shown by the production rate of p-nitrophenol in TN5 cells. Figure 3B shows the results of sialic acid detected in recombinant boIFN-τ. Figure 3C shows the results of expressed recombinant boIFN-τ detected by CBB staining. Lanes 1 to 6 in the figures show the results obtained at addition amounts of 2-ADN, 0, 0.05, 0.5, 1, 2 and 5 mM, respectively.

As shown in Figure 3A, the GlcNcase activity was inhibited by 2-ADN in a concentration-dependent manner. About 80% of the activity was inhibited by 5 mM 2-ADN.

As shown in the results of the lectin blot analysis (Figure 3B) and the CBB staining (Figure 3C), the SNA-reactive band intensity increased by adding 2-ADN in a concentration dependent manner. However, the total amount of protein of each lane is the same.

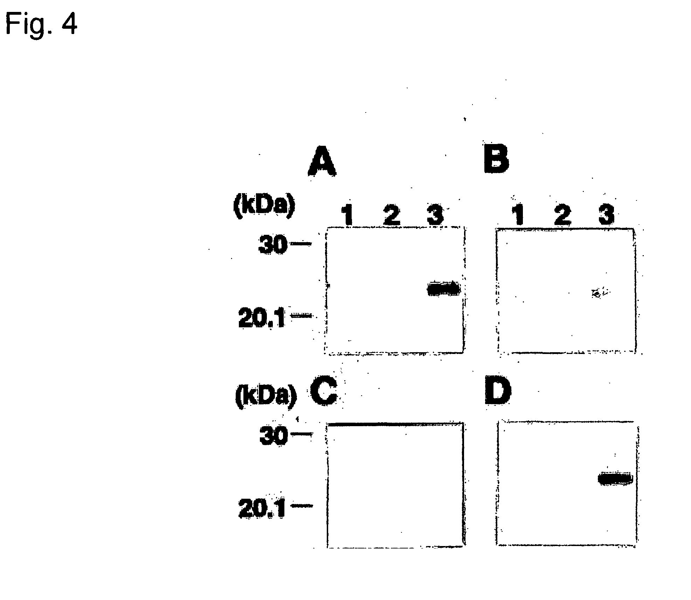

Furthermore, to confirm the α-2,6-linked sialylation of N-glycan of recombinant boIFN-τ, the following two experiments were performed. First, the membrane on which the protein was transferred was treated with sialidase (derived from *Arthobacter ureafaciens,* manufactured by Roche Molecular Biochemicals) and subjected to the lectin blot analysis. Then, SNA was preincubated with 10 mM 3'-sialyllactose or 0.1 mM 6'-sialyllactose and subjected to the lectin blot analysis. The results are shown in Figure 4. The samples used in Figure 4 are as follows: Lane 1 is the culture supernatant of TN5 cells infected with a wild-type virus cultured in the presence of 2-ADN; Lane 2 is the culture supernatant containing recombinant boIFN-τ obtained from the culture in the absence of 2-AND (5 mM); and Lane 3 is the culture supernatant containing recombinant boIFN-τ obtained from the culture in the presence of 2-ADN. Figure 4A shows the results where siliadase treatment was not performed and Figure 4B shows the results where siliadase treatment was performed. As shown in the figures, when the sample is treated with siliadase, the band of recombinant boIFN-τ expressed by TN5 cells in the presence of 2-ADN disappeared. Figure 4D shows the results where preincubation was performed with 10 mM 3'-sialyllactose and Figure 4C shows the results where preincubation was performed with 0.1 mM 6'-sialyllactose. As shown in the figures, when the sample is treated with 6'-sialyllactose, the band of recombinant boIFN-τ in the presence of 2-ADN disappeared. On the other hand, the band of recombinant boIFN-τ having SNA combined therewith treated with 3'-sialyllactose and Maackia amurensis agglutinin lectin appeared. Maackia amurensis agglutinin lectin recognizes α-2,3-linked sialic acid and does not react with recombinant boIFN-τ. These results demonstrate that not α-2,3-linked sialic acid but α-2,6-linked sialic acid is added to the N-glycan terminus of recombinant boIFN-τ under the conditions inhibiting GlcNAcase.

### [Example 3] Study on recombinant granulocyte-macrophage colony-stimulating factor (GM-CSF) and interleukin 2 (IL2)

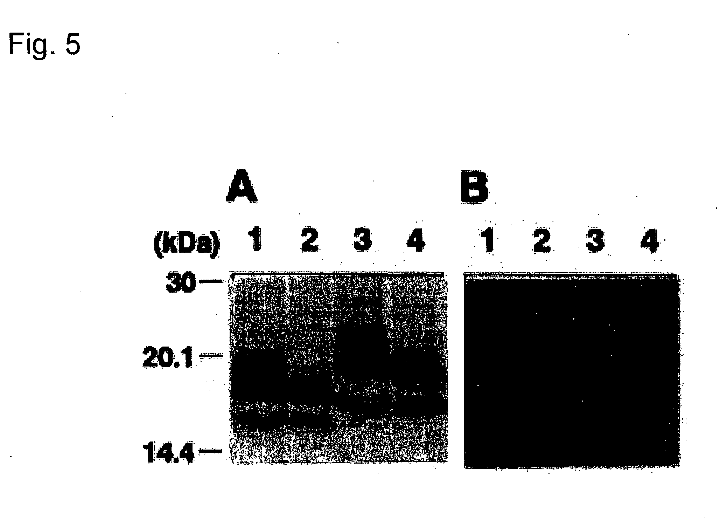

Whether or not sialylation of other glycoproteins occurs was investigated. Similarly to the case of IFN-τ, TN5 cells having a bovine CM-GSF gene or a swine IL2 gene were prepared and cultured. The supernatant of the culture was subjected to lectin blot analysis. The results are shown in Figure 5. Figure 5A shows the results of CBB staining and Figure 5B shows the results of lectin blot analysis. Samples of individual lanes are as follows: Lanes 1 and 2 are recombinant GM-CSFs obtained in the presence and absence of 2-ADN, respectively; and Lanes 3 and 4 are recombinant IL2 obtained in the presence and absence of 2-ADN, respectively. As shown in the figures, the molecular weight of recombinant GM-CSF and recombinant IL2 obtained in the presence of 2-ADN were significantly larger than those obtained in the absence of them. In addition, the lectin blot analysis demonstrates that recombinant GM-CSF and recombinant IL2 can be reacted with SNA. These results demonstrate that when the GlcNAcase activity is inhibited for recombinant GM-CSF and recombinant IL2, α-2,6-linked sialylation of the terminal occurs.

### Industrial Applicability

As shown in Examples, when a recombinant glycoprotein is obtained by culturing insect cells having a gene encoding the glycoprotein while the GlcNAcase activity is suppressed, inhibited, or eliminated, sialic acid is bound to the sugar chain of the recombinant protein. In short, a mammalian type recombinant protein was obtained. Accordingly, the present invention enables to express a recombinant protein having sialic acid added thereto, which is more stable, close to a protein inherent in a mammal, and suitable for administration to animals, in insect cells in a large amount. Thus, the present invention can greatly contribute to development of pharmaceuticals for animals and humans.

For all publications cited in this specification, the entire contents of which are incorporated herein. Furthermore, those skilled in the art may easily understand that various modifications and changes may be made without departing from the technical idea and scope of the present invention recited in the scope of the claims attached hereto. The present invention contemplates including such modifications and changes.

## Claims

1. A method of expressing a recombinant protein having a sugar chain with sialic acid added thereto in an insect or insect cells, which comprises suppressing, inhibiting or eliminating N-acetylglucosaminidase activity contained in the insect cells.

2. The method according to claim 1, wherein the insect is silkworm.

3. A method of producing a recombinant protein having a sugar chain with sialic acid according to claim 1 added thereto, which comprises suppressing, inhibiting or eliminating N-acetylglucosaminidase activity contained in insect cells, culturing the insect cells infected with a baculovirus having a mammal-derived protein gene introduced therein to express the protein.

4. The method according to any one of claims 1 to 3, wherein the suppressing, inhibiting or eliminating N-acetylglucosaminidase activity is performed by addition of an N-acetylglucosaminidase inhibitor.

5. The method according to claim 4, which comprises pretreating insect cells with an N-acetylglucosaminidase inhibitor before the insect cells are infected with a baculovirus having a mammal-derived protein gene introduced therein.

6. The method according to claim 4 or 5, wherein the N-acetylglucosaminidase inhibitor is 2-acetoamide-1,2-dideoxynojirimycin.

7. The method according to any one of claims 1 to 6, wherein the protein is selected from the group consisting of interferons, granulocyte-macrophage colony-stimulating factors, and interleukins.

8. The method according to claim 7, wherein the protein is interferon-τ.
